(19) Europäisches Patentamt
European Patent Office
Office européen des brevets

(11) EP 4 691 499 A1

(12) EUROPEAN PATENT APPLICATION
published in accordance with Art. 153(4) EPC

(43) Date of publication:
**11.02.2026 Bulletin 2026/07**

(21) Application number: **24780250.7**

(22) Date of filing: **26.03.2024**

(51) International Patent Classification (IPC):
***A61K 47/69*** *(2017.01)* ***A61K 9/10*** *(2006.01)*
***A61K 31/52*** *(2006.01)* ***A61K 31/513*** *(2006.01)*
***A61K 31/522*** *(2006.01)* ***A61K 31/7072*** *(2006.01)*
***A61K 47/58*** *(2017.01)* ***A61K 48/00*** *(2006.01)*
***A61P 27/02*** *(2006.01)* ***A61P 29/00*** *(2006.01)*
***A61P 31/12*** *(2006.01)* ***C08F 8/40*** *(2006.01)*
***C08F 20/22*** *(2006.01)*

(52) Cooperative Patent Classification (CPC):
**A61K 9/10; A61K 31/513; A61K 31/52; A61K 31/522; A61K 31/7072; A61K 47/58; A61K 47/69; A61K 48/00; A61P 27/02; A61P 29/00; A61P 31/12; C08F 8/40; C08F 20/22**

(86) International application number:
**PCT/JP2024/011836**

(87) International publication number:
**WO 2024/204137 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053567**

(71) Applicant: **Seed Co., Ltd.**
**Tokyo 113-8402 (JP)**

(72) Inventors:
- **YOSHIMURA, Yuichi**
  **Sendai-shi, Miyagi 981-8558 (JP)**
- **SAITO, Yukako**
  **Sendai-shi, Miyagi 981-8558 (JP)**
- **YAMAZAKI, Yoshiko**
  **Tokyo 113-8402 (JP)**
- **OTAKE, Hideyuki**
  **Tokyo 113-8402 (JP)**

(74) Representative: **Schulz Junghans Patentanwälte PartGmbB**
**Großbeerenstraße 71**
**10963 Berlin (DE)**

# (54) HYDROGEL WITH DRUG BOUND THERETO

(57) The objective of the present invention is to provide a transparent hydrogel binding a poorly water-soluble drug to the surface and/or inside. The objective is solved by a drug-bound hydrogel, wherein the drug is at least one drug selected from the group consisting of nucleic acid and nucleic acid analog, and wherein the drug is bound to at least one portion selected from the group consisting of the surface portion and interior portion of base material of the hydrogel via an amphiphilic linker.

Figure 1

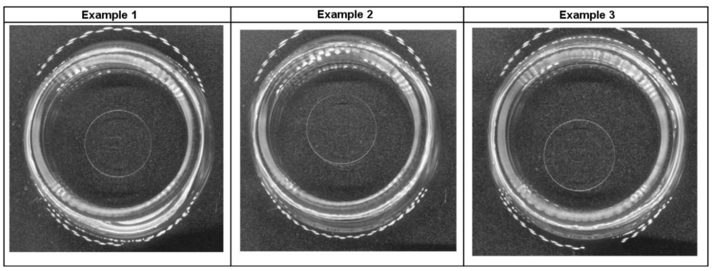


EP 4 691 499 A1

## Description

### Technical Field

The present invention relates to a drug-bound hydrogel in which a drug is bound to a base material of the hydrogel.

### Background Art

Nucleic acid drugs are a class of drugs based on a basic framework of natural nucleic acid or chemically modified nucleic acid, and specifically act on a target protein or gene to control the function of them and treat a disease.

Among the nucleic acid drugs, idoxuridine, which is a uridine analog, is an anti-herpesvirus drug used as an ophthalmic solution to treat herpetic keratitis. In recent years, eye drops for feline herpes containing idoxuridine as the active ingredient have been marketed and used in the veterinary ophthalmology field. However, the eye drops containing idoxuridine as the active ingredient are administered every 1 to 2 hours, thereby placing a heavy burden on the patient due to the frequent administration.

On the other hand, if the patient is a contact lens wearer, the ophthalmic solution may have adverse effects on the contact lens, such as distortion and alteration. In addition, there is a risk that preservatives commonly used in commercially available eye drops may be taken inside the contact lens and cause intraocular allergies in the patient. Therefore, the drug treatment with the ophthalmic solution is not suitable for the contact lens wearer.

Therefore, a safe and easy way for the contact lens wearer to receive the drug treatment is to use drug delivery system (DDS). In the DDS method, the patient wears a contact lens configured to contain a therapeutic drug in advance, thereby contacting or releasing the drug into the ocular tissue to treat the patient.

As a polymer gel for DDS which is available for a contact lens to achieve DDS, there is a known hydrogel in which the molar ratio of anionic monomers and cationic monomers, which are components of the hydrogel, is unbalanced to ionically bind an anionic drug to the excess cationic monomers of the hydrogel (see, for example, Patent Document 1).

There is also a known hydrogel which uses a condensation product, or salt thereof, of an alkyl quaternary ammonium compound with substituted or unsubstituted aralkyl groups and (meth)acrylic acid, which is a cationic monomer as a component of the hydrogel and is capable of holding the anionic drug, thereby increasing the amount of drug incorporated and decreasing the adverse effects on gel strength and shape stability after drug release (for example, see Patent Document 2).

### Citation List

#### Patent Documents

Patent Document 1: JP 2004-307574 A
Patent Document 2: WO 2015/159942 A

### Summary of the Invention

#### Problems to be Solved by the Invention

A hydrogel is formed by swelling a polymer with water. However, with regard to the hydrogel for DDS disclosed in Patent Document 1 or 2, if the drug to be bound is poorly water-soluble, it is very difficult to bind the drug to the hydrogel. Even if the drug could be bound, the resulting hydrogel would have the problem of cloudiness due to phase separation.

In view of the above circumstances, it is an objective of the present invention to provide a transparent hydrogel binding a poorly water-soluble drug to the surface and/or inside.

#### Means of Solving the Problems

In order to solve the above-identified problem, the present inventors attempted to directly bind a poorly water-soluble drug to a polymer that constitutes a hydrogel (hydrogel base material). However, the present inventors have found it very difficult to achieve such direct binding because the hydrogel base material contains hydrophilic portions serving for water swelling.

As a result of extensive efforts, the present inventors have come to have an idea of binding a poorly water-soluble

drug to a hydrogel base material via an amphiphilic linker. As a result of the further trial and error process, the present inventors surprisingly found that it was possible to obtain a highly transparent hydrogel that contains a poorly water-soluble drug bound to the surface and/or interior by binding the poorly water-soluble drug to an amphiphilic linker to obtain a complex followed by binding the complex to the hydrogel base material.

Finally, based on such findings, the present inventors have succeeded in inventing a transparent hydrogel containing a poorly water-soluble drug bound to the surface and/or interior as a solution to the problem of the present invention. As such, the present invention has been completed on the basis of the findings and successful examples that were first found or obtained by the present inventors.

According to the present invention, the following aspects are provided:

[1] A drug-bound hydrogel, wherein the drug is at least one drug selected from the group consisting of nucleic acid and nucleic acid analog, and wherein the drug is bound to at least one portion selected from the group consisting of the surface portion and interior portion of base material of the hydrogel via an amphiphilic linker.

[2] The drug-bound hydrogel according to claim 1, wherein the amphiphilic linker is a phosphate linker represented by the following general formula (I)

[Chemistry 1]

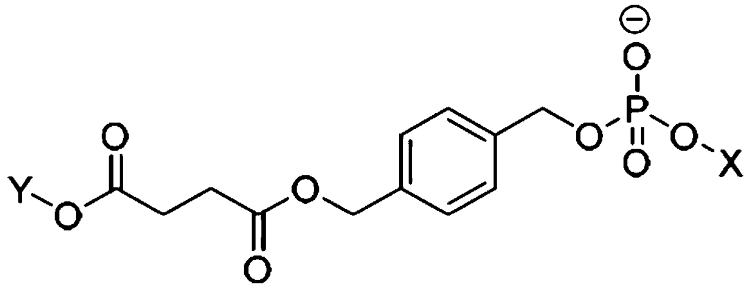

(I)

(wherein X represents a drug binding site and Y represents a hydrogel base material binding site)
, or
a phosphate linker represented by the following general formula (II)

[Chemistry 2]

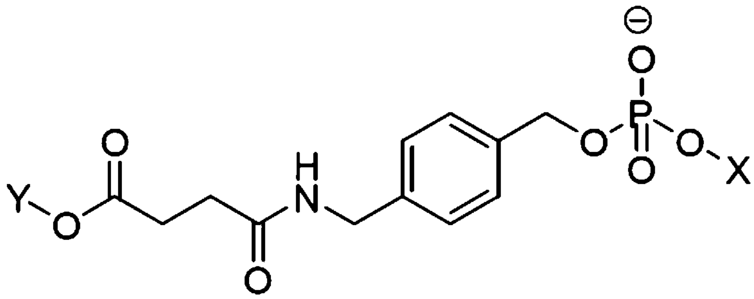

(II)

(wherein X represents a drug binding site and Y represents a hydrogel base material binding site).

[3] The drug-bound hydrogel according to claim 1 or 2, wherein the drug is at least one nucleic acid analog selected from the group consisting of idoxuridine, acyclovir, abacavir, emtricitabine, zidovudine and lamivudine.

Effects of the Invention

According to the present invention, there can be provided a transparent hydrogel having a drug with low solubility in water bound on the surface and/or inside. In addition, the poorly water-soluble drug is bound to the hydrogel via an amphiphilic linker, so that the drug can be contacted or released into the ocular tissue while decreasing the adverse effects on the hydrogel base material since there is a certain distance between the drug's degradation point (binding site) and the hydrogel base material.

Thus, the drug-bound hydrogel according to one embodiment of the present invention is expected to be applied to ocular lenses such as a contact lens being a transparent hydrogel for DDS that allows a poorly water-soluble drug to be contacted or released into the ocular tissue.

Brief Description of the Drawing

Figure 1 is photographs obtained by taking the idoxuridine-bound hydrogels of Examples 1 to 3 from the top surface against a black background, as described in Examples below.

Description of Embodiments

While each aspect that forms one embodiment of the present invention will now be described in detail, the present invention is not limited only by the matters of this section, and may take various forms to the extent that its objective can be achieved.

Unless otherwise specified, each term used herein is used in the meaning commonly used by those skilled in the art, in particular in the chemical fields relating to hydrogels, ocular lenses, drugs and others, and should not be construed to have any meaning that is unduly limiting. Also, any speculations and theories herein are made on the basis of the knowledge and experiences of the present inventors and as such, the present invention is not bound by any such speculations and theories.

The term "poorly water-soluble" refers to the degree of solubility in water classified as "Sparingly soluble" to "Practically insoluble, or insoluble" in the section "General Notices" in Japanese Pharmacopoeia, 18th Edition.

The term "content" is synonymous with concentration and amount used (amount added), and means the ratio of the amount of a component relative to the total amount of the final product. However, the total content of the components may not exceed 100%. Unless otherwise specified, the unit of content used herein means "% by mass" and "wt%". If a commercial product is employed, the content of a component is preferably the amount of the component contained in the commercial product, but the content may also be the amount of the commercial product itself.

The term "and/or" as used herein means either any one of, any combination of two or more of, or combination of all of listed related items.

The terms "include," "comprise," and "contain" mean that an element(s) other than an element(s) as explicitly indicated can be added as inclusions, which are, for example, synonymous with "at least include," but encompasses the meaning of "consist of" and "substantially consist of". In other words, the terms may mean, for example, to include an element(s) as explicitly indicated as well as any one element or any two or more elements, to consist of an element(s) as explicitly indicated, or substantially consist of an element(s) as explicitly indicated. Such elements include limitations such as components, steps, conditions, and parameters.

The wording "to" for indicating a range of values is a range that include values preceding and following the wording, and also includes a range excluding any one of the lower and upper limits included in the range. For example, "0% to 100%" means a range of values more than or equal to 0%, of values less than or equal to 100%, and of values between 0% and 100%. The terms "more than" and "less than" used herein means the lower and upper limits without including a value following the term, respectively. For example, "more than 1" means a value beyond 1, and "less than 100" means a value below 100. The term "about" means an amount within $\pm 10\%$ of the quantity following the term. For example, "about 100" means $100 \pm 10\%$, i.e., 90 to 110.

The number of digits of an integer equals to its significant figure. For example, 1 has one significant figure and 10 has two significant figures. For a decimal number, the number of digits after a decimal point equals to its significant figure. For example, 0.1 has one significant figure and 0.10 has two significant figures.

The hydrogel according to one embodiment of the present invention can bring the bound drug into contact with the portion the hydrogel contacted. In addition, the hydrogel according to one embodiment of the present invention is capable of gradually releasing the bound drug, i.e., sustained release, by undergoing hydrolysis or other reactions. One specific embodiment of the hydrogel is a drug-bound hydrogel that can be placed in the eye. The hydrogel according to one embodiment of the present invention is mainly composed of a drug and a hydrogel.

[Drug]

The drug is nucleic acid, nucleic acid analog, or both of them. The nucleic acid normally means a polymer (such as DNA and RNA) in which two or more nucleotides are linked by phosphodiester linkage. When used herein, the nucleic acid may include a nucleotide as well as a nucleoside and a nucleobase (such as a purine base and a pyrimidine base), which are a portion of nucleotide, in addition to the above-mentioned polymer. The nucleic acid analog may be any compound that has a structure similar to the nucleic acid.

Examples of the nucleic acid and the nucleic acid analog include nucleobases such as adenine, cytosine, guanine, thymine and uracil; modified nucleobases such as 5-methylcytosine, pseudouridine, dihydrouridine, inosine, 7-methylguanosine; and nucleic acid analogs such as idoxuridine, acyclovir, valacyclovir, abacavir, emtricitabine, zidovudine, lamivudine, adefovir, entecavir, tenofovir, cidofovir and vidarabine. In view of eye treatment, the drug is preferably the nucleic acid analog, and more preferably idoxuridine, which is used as an anti-herpesvirus drug, and acyclovir, which is used as a pharmaceutical drug for treating infections caused by herpes simplex virus and varicella-zoster virus.

The present inventors have found that it is almost impossible to bind the drugs such as idoxuridine and acyclovir to the polymer (hydrogel base material) that constitutes a hydrogel having hydrophilic portions since the drugs have poor solubility in water. In the hydrogel according to one embodiment of the present invention, the drug is bound to the hydrogel base material via an amphiphilic linker. The amount of the drug contained in the hydrogel according to one embodiment of the present invention is equal to or less than the amount of the amphiphilic linker bound to the hydrogel according to one embodiment of the present invention.

[Amphiphilic linker]

The amphiphilic linker has a structure in which there are a hydrophilic portion and a hydrophobic portion in the same molecule, and each of the hydrophilic and hydrophobic portions has a binding site to other molecules.

The amphiphilic linker preferably has a structure in which the distance between the hydrogel base material binding site and the drug binding site is within a certain distance. Such a certain distance is expected to suppress the adverse effects on the hydrogel base material caused by drug detachment reaction in releasing the drug. In view of such an aspect, the amphiphilic linker is preferably a phosphate linker represented by the following general formula (I)

[Chemistry 3]

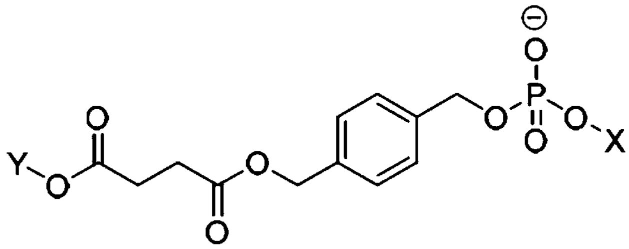

(I)

, and
a phosphate linker represented by the following general formula (II)

[Chemistry 4]

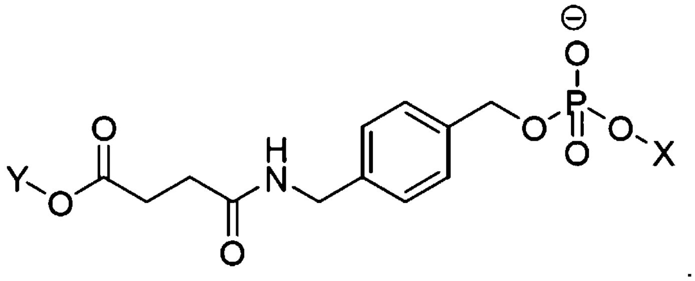

(II)

In the general formulas (I) and (II), X represents a drug binding site and Y represents a hydrogel base material binding site.

The method of obtaining the amphiphilic linker is not particularly limited. Examples of the method include the use of chemical synthesis and commercially available products. The method of synthesizing the amphiphilic linker is not particularly limited. Examples of the method include preferably a method including binding one end of the amphiphilic linker to the drug and then converting the other end of the amphiphilic linker to a hydrophilic portion to obtain a drug-bound amphiphilic linker.

[Method for synthesis of drug-bound amphiphilic linker]

Examples of the starting material constituting the hydrophobic portion of the phosphate linker according to the general formula (I) or (II) include compounds according to the following formulas (III) and (IV). In the formulas, "DMTr" represents 4,4'-dimethoxytriphenylmethyl group, "Boc" represents tert-butoxycarbonyl group, and "iPr" represents isopropyl group.

[Chemistry 5]

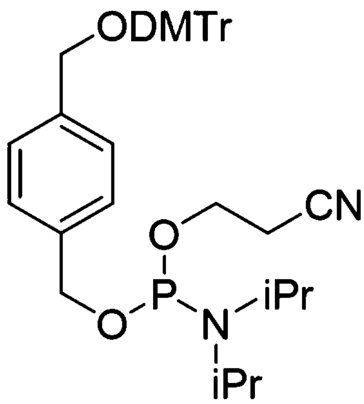

(III)

[Chemistry 6]

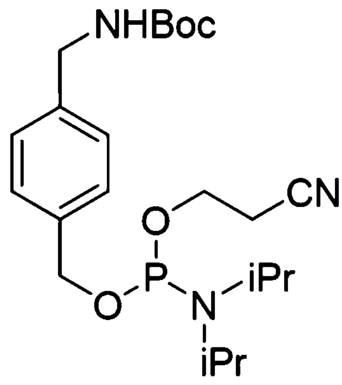

(IV)

Specific examples of the method of producing the drug-bound amphiphilic linker include the following method of producing the drug-bound linker (V) with idoxuridine bound to the phosphate linker according to the general formula (I).

Scheme (1) shows the scheme for production of the drug-bound linker (V) using idoxuridine and compound (III). Scheme (1) includes reacting idoxuridine and compound (III) with 5-(ethylthio)-1H-tetrazole followed by tert-butylhydroperoxide (TBHP) to obtain compound (III)-A, subjecting the protecting group of compound (III)-A to deprotection to obtain compound (III)-B, reacting compound (III)-B with succinic anhydride in the presence of pyridine to obtain compound (III)-C, subjecting compound (III)-C to deprotection, and then reacting the deprotected compound (III)-C with tetrabutylammonium fluoride (TBAF) to obtain the drug-bound linker (V) having the structure with idoxuridine bound to one end of compound (III) and with succinic acid bound to the other end of compound (III).

[Chemistry 7]

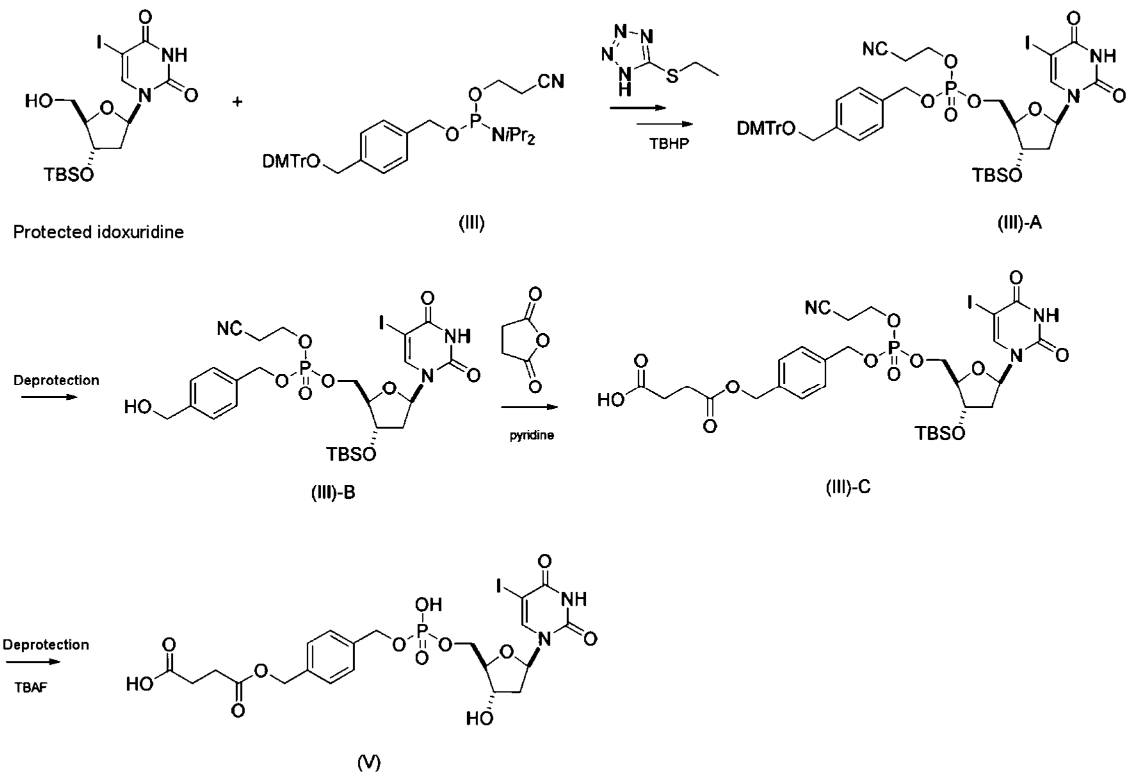

Protected idoxuridine
+
(III)
(III)-A
(III)-B
(III)-C
(V)

Scheme (1)

Specific examples of the method of producing the drug-bound amphiphilic linker also include the following

method of producing the drug-bound linkers (VI) and (VII) with acyclovir bound to the phosphate linkers according to the general formulas (I) and (II), respectively.

Scheme (2) shows the scheme for production of the drug-bound linker (VI) using acyclovir and compound (III). Scheme (2) includes using compound (III) and acyclovir as starting materials to obtain compound (III)-D and compound (III)-E followed by the drug-bound linker (VI) having the structure with acyclovir bound to one end of compound (III) and with succinic acid bound to the other end of compound (III), as with Scheme (1).

[Chemistry 8]

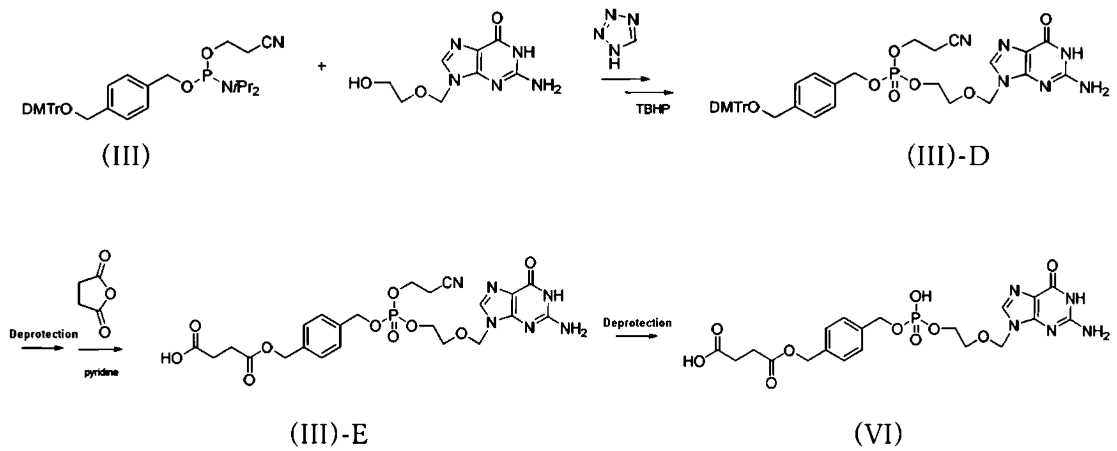

(III) (III)-D
(III)-E (VI)

Scheme (2)

Scheme (3) shows the scheme for production of the drug-bound linker (VII) using acyclovir and compound (IV). Scheme (3) includes using compound (IV) and acyclovir as starting materials to obtain compound (IV)-A and compound (IV)-B followed by the drug-bound linker (VII) having the structure with acyclovir bound to one end of compound (IV) and with succinic acid bound to the other end of compound (IV), as with Scheme (1).

[Chemistry 9]

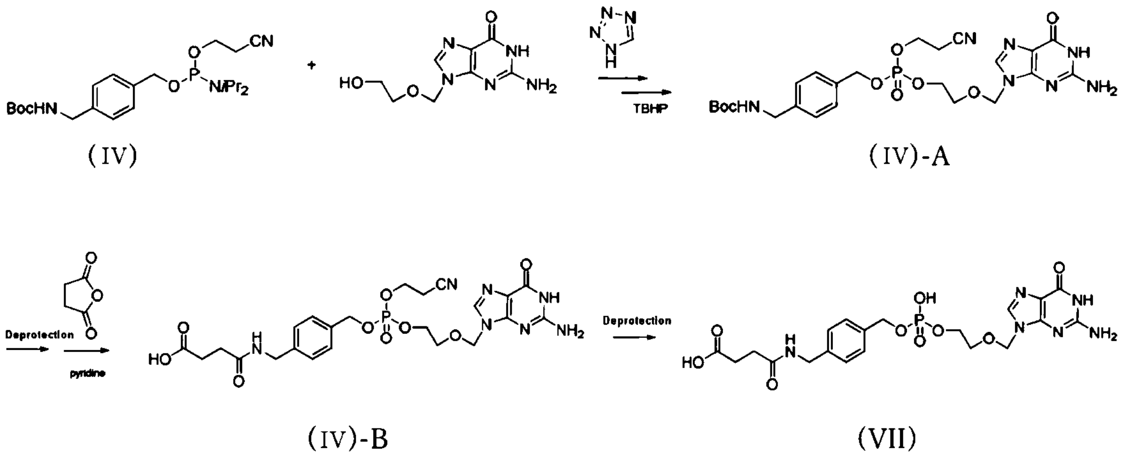

(IV) (IV)-A
(IV)-B (VII)

Scheme (3)

The drug-bound linkers (V) to (VII) bind to the hydrogel base material at the carboxyl group (-COOH) present in the end. For example, the drug-bound linkers (V) to (VII) may bind to the hydrogel base material through dehydration-condensation reaction between the carboxyl group present in the end and the amino group ($-NH_2$), carboxyl group or other group on the hydrogel base material.

[Drug-bound hydrogel]

The hydrogel is based on a polymerized compound (polymer) of monomer components including a hydrophilic monomer. The hydrogel is swollen by an aqueous solution such as water or a water-containing solution, and retains the aqueous solution in the polymer matrix. When used herein, the polymer itself, excluding the aqueous solution, is referred to as the hydrogel base material. The polymer (polymerized compound) is produced by polymerization of monomers, and the constituent unit derived from each monomer in the polymer is also referred to as a portion or residue.

Specific examples of the hydrogel include hydrogels produced with hydrophilic monomers, hydrogels produced with hydrophilic monomers and monomers copolymerizable with the hydrophilic monomers, such as hydrophobic monomers and crosslinking monomers.

The drug-bound hydrogel according to one embodiment of the present invention has a moiety that can bind to the amphiphilic linker. Thus, the drug-bound hydrogel according to one embodiment of the present invention has a portion with a moiety that can bind to the amphiphilic linker. Such a portion with a moiety that can bind to the amphiphilic linker may be either a portion from a hydrophilic monomer or a portion from a monomer other than a hydrophilic monomer.

If the hydrogel base material binding site in the amphiphilic linker is a carboxyl group, then such a portion with a moiety that can bind to the amphiphilic linker present in the drug-bound hydrogel according to one embodiment of the present invention is, for example, preferably a portion from an amino group-containing monomer and/or a carboxyl group-containing monomer.

The amino group-containing monomer is not particularly limited, as long as the monomer is a polymerizable compound having in the molecule at least one amino group and at least one polymerizable group such as (meth)acryloyl group and vinyl group. Examples of the amino group-containing monomer include N-(2-aminoethyl)methacrylamide hydrochloride, N-methacryloyl ethylenediamine hydrochloride, 2-aminoethyl methacrylate hydrochloride, N-(3-aminopropyl) methacrylamide hydrochloride, aminomethyl (meth)acrylate, 4-aminobutyl (meth)acrylate, 2-aminopropyl (meth) acrylate, 5-aminopentyl (meth)acrylate, 2-amino-1-methylethyl (meth)acrylate, 6-aminohexyl (meth)acrylate, 2-aminobutyl (meth)acrylate, 1-aminoethyl (meth)acrylate, 5-amino-1-methylpentyl (meth)acrylate, 1-aminobutyl (meth)acrylate, 2-amino-2-methylpropyl (meth)acrylate, 1-aminohexyl (meth)acrylate, 11-aminoundecyl (meth)acrylate, 3-amino-3-methylbutyl (meth)acrylate, 2-amino-1,1-dimethylpropyl (meth)acrylate, 12-amino-dodecyl (meth)acrylate, 1-amino 1-methylethyl (meth)acrylate, 2-amino-2-methylhexyl (meth)acrylate, 7-amino-3,4-dimethyloctyl (meth)acrylate, 7-amino-3,7-dimethyloctyl (meth)acrylate, 8-amino-4,8-dimethylnonyl(meth)acrylate, 6-amino-2,4-hexadiyne(meth)acrylate, 12-amino-5,7-dodecadiyne(meth)acrylate, 2-aminoethyl vinyl ether, 3-aminopropyl vinyl ether, 1-aminoethyl vinyl ether, 4-aminobutyl vinyl ether, 2-aminopropyl vinyl ether, 1-aminopentyl vinyl ether, 2-aminobutyl vinyl ether, 1-aminopropyl vinyl ether, 1-amino-3-methylhexyl vinyl ether, 2-amino-2-methylpropyl vinyl ether, and 2-amino-2,6-dimethyloctyl vinyl ether, preferably N-(2-aminoethyl) methacrylamide hydrochloride, N-methacryloylethylenediamine hydrochloride, 2-aminoethyl methacrylate hydrochloride, and N-(3-aminopropyl)methacrylamide hydrochloride. The amino group-containing monomer may be used either individually or in combination of two or more of those listed above. (Meth)acrylate is a generic term for acrylate and methacrylate. The same applies for (meth)acrylic acid and (meth)acryloyl group.

The carboxyl group-containing monomer is not particularly limited, as long as the monomer is a polymerizable compound having in the molecule at least one carboxyl group and at least one polymerizable group such as (meth)acryloyl group and vinyl group. Examples of the carboxyl group-containing monomer include methacrylic acid, methacryloxyethyl succinate, (meth)acrylic acid 2-carboxyethyl, (meth)acrylic acid carboxymethyl, (meth)acrylic acid 3-carboxypropyl, (meth)acrylic acid 1-carboxyethyl, (meth)acrylic acid 4-carboxybutyl, 2-carboxypropyl (meth)acrylate, 5-carboxypentyl (meth)acrylate, 6-carboxyhexyl (meth)acrylate, 2-carboxy-2-dimethylhexyl (meth)acrylate, 2-carboxy-1-dimethylhexyl (meth)acrylate, 5-carboxyheptyl (meth)acrylate, 11-carboxyundecyl (meth)acrylate and 12-carboxydodecyl (meth)acrylate, preferably methacrylic acid and methacryloxyethylsuccinate. The carboxyl group-containing monomer may be used either individually or in combination of two or more of those listed above.

In the hydrogel base material, the portion with a moiety that can bind to the amphiphilic linker becomes the binding point for the amphiphilic linker, and the amount of the portion correlates with the drug content in the drug-bound hydrogel according to one embodiment of the present invention. Therefore, the content of the monomer providing the portion with a moiety that can bind to the amphiphilic linker such as the amino group-containing monomer and carboxyl group-containing monomer (hereinafter referred to as amphiphilic linker-binding monomer) may be adjusted appropriately based on the desired content of the drug to be bound, and is not particularly limited. Examples of the content include preferably from 1 wt% to 15 wt%, more preferably from 1 wt% to 10 wt%, and still more preferably from 1 wt% to 5 wt%, when the drug-bound hydrogel is applied to an ocular contact lens and the optical and physicochemical properties of the resulting contact lens are taken into consideration.

When the amphiphilic linker-binding monomer has a hydrophilic group, the monomer may be used as a hydrophilic monomer. On the other hand, when the amphiphilic linker-binding monomer has no hydrophilic group, a hydrophilic monomer is used as a second monomer component separately from the above monomer. Even when the amphiphilic linker-binding monomer is used as a hydrophilic monomer, the other hydrophilic monomer may be used in

combination.

The hydrophilic monomer is not particularly limited, as long as the monomer is a polymerizable compound that has in the molecule at least one hydrophilic group and at least one polymerizable group such as (meth)acryloyl group and vinyl group. Examples of the monomer include hydroxymethyl (meth)acrylate, 2-hydroxyethyl (meth)acrylate, 2-hydroxypropyl (meth)acrylate, 2,3-dihydroxypropyl (meth)acrylate, 2-polyethylene glycol mono(meth)acrylate, 2-polypropylene glycol (meth)acrylate, N,N-dimethyl (meth)acrylamide, N,N-diethyl (meth)acrylamide and N-vinylpyrrolidone, preferably 2-hydroxyethyl methacrylate. The hydrophilic monomer may be used either individually or in combination of two or more of those listed above.

The content of the hydrophilic monomer is not particularly limited, as long as the content is an amount to the extent that the polymer obtained by copolymerization of monomer components including the hydrophilic monomer is capable of forming a hydrogel. Examples of the content include preferably 50 wt% to 99 wt%, more preferably 60 wt% to 98 wt%, and still more preferably 70 wt% to 96 wt%, relative to the total amount of monomer components. When the content of the hydrophilic monomer is less than 50 wt%, the resulting hydrogel may become less flexible and have the impaired wearing feeling. When the content of the hydrophilic monomer is more than 99 wt%, the resulting hydrogel may not bind a sufficient amount of drug since the content of the amphiphilic linker-binding monomer may relatively decrease.

When the drug-bound hydrogel according to one embodiment of the present invention is used as an ocular hydrogel or the like, the hydrogel may contain a hydrophobic monomer as a monomer component in order to adjust the optical and physicochemical properties.

The hydrophobic monomer is not particularly limited, as long as the monomer is a polymerizable compound that has no hydrophilic group and has a polymerizable group such as (meth)acryloyl group and vinyl group in the molecule. Examples of the monomer include (meth)acrylic monomers such as trifluoroethyl methacrylate, methacrylamide, siloxanyl methacrylate, methyl methacrylate, n-butyl methacrylate, tert-butyl methacrylate, benzyl methacrylate, ethylhexyl methacrylate and lauryl (meth)acrylate; and silicone-containing monomers such as α-mono(methacryloyloxymethyl) polydimethylsiloxane, α,ω-di(methacryloyloxymethyl) polydimethylsiloxane, α-mono(3-methacryloyloxypropyl) polydimethylsiloxane, α,ω-di(3-methacryloyloxypropyl) polydimethylsiloxane, α-mono(3-methacryloyloxybutyl) polydimethylsiloxane, α,ω-di(3-methacryloyloxybutyl) polydimethylsiloxane, α-monovinyl polydimethylsiloxane, α,ω-divinyl polydimethylsiloxane, 3-tris(trimethylsiloxy)silylmethyl (meth)acrylate, 3-tris(trimethylsiloxy)silylpropyl (meth)acrylate, 3-methylbis(trimethylsiloxy)silylmethyl (meth)acrylate, 3-methylbis(trimethylsiloxy)silylpropyl (meth)acrylate, 3-trimethylsiloxydimethylsilylmethyl (meth)acrylate, 3-trimethylsiloxydimethylsilylpropyl (meth)acrylate and 3-methyldimethoxysilylpropyl (meth)acrylate. The hydrophobic monomer may be used either individually or in combination of two or more of those listed above.

The content of the hydrophobic monomer may be adjusted appropriately depending on the desired optical and physicochemical properties to be imparted to the hydrogel, and is not particularly limited. Examples of the content include preferably 0 wt% to 20 wt%, and more preferably 0 wt% to 10 wt%, relative to the total amount of monomer components. When the content of the hydrophobic monomer is more than 20 wt%, the resulting hydrogel may become less flexible and have the impaired wearing feeling.

The drug-bound hydrogel according to one embodiment of the present invention may contain a cross-linking monomer as a monomer component in order to control the formation of the network structure of the hydrogel and the mechanical strength of the hydrogel.

The cross-linking monomer is not particularly limited, as long as the monomer is a polymerizable compound that has two or more polymerizable groups such as (meth)acryloyl group and vinyl group in the molecule. Examples of the monomer include ethylene glycol di(meth)acrylate, methylene bis(meth)acrylamide, 2-hydroxy-1,3-di(meth)acryloxypropane and trimethylolpropane tri(meth)acrylate, preferably ethylene glycol di(meth)acrylate. The cross-linking monomer may be used either individually or in combination of two or more of those listed above.

The content of the cross-linking monomer may be set appropriately according to the desired network structure formation and mechanical strength to be imparted to the hydrogel, and is not particularly limited. Examples of the content include preferably 0.1 wt% to 5 wt%, and more preferably 0.3 wt% to 3 wt%, relative to the total amount of monomer components. When the content of the cross-linking monomer is more than 5 wt%, the resulting hydrogel may become less flexible and have the impaired wearing feeling, thereby being not suitable for an ocular hydrogel.

In the drug-bound hydrogel according to one embodiment of the present invention, the drug is bound via the amphiphilic linker to the portion derived from the amphiphilic linker-binding monomer on the surface, interior and both thereof present in the hydrogel base material. As long as the drug-bound hydrogel according to one embodiment of the present invention has such a structure, the production method is not particularly limited.

The drug-bound hydrogel according to one embodiment of the present invention may be produced by a combination of steps known to those skilled in the art. The drug-bound hydrogel according to one embodiment of the present invention may be produced, for example, by the production method including the following steps (1) to (4):

(1) agitating and dissolving a mixture of monomer components composed of monomers constituting a copolymer as

the base material of the drug-bound hydrogel and a polymerization initiator to obtain a monomer mixture solution;
(2) placing the resulting monomer mixture solution into a desired molding die and subjecting the molding die to copolymerization reaction to obtain a copolymer;
(3) detaching the obtained copolymer from the molding die cooled after the copolymerization reaction, cutting and/or polishing the copolymer as necessary, and then hydrating and swelling the copolymer to obtain a hydrogel; and
(4) subjecting the reaction solution containing the obtained hydrogel and the drug-bound amphiphilic linker and, if necessary, a dehydration condensation agent to dehydration condensation reaction to obtain a drug-bound hydrogel in which the drug is bound to the surface and/or interior of base material of the hydrogel via the amphiphilic linker.

In the step (1) of obtaining the monomer mixture solution, the mixture of monomer components and the polymerization initiator may be stirred and dissolved according to the usual method. For example, the monomer mixture solution may be prepared by stirring and mixing the monomer components in an amount set to impart the desired physical properties to the resulting copolymer and the polymerization initiator with use of a magnetic stirrer or other stirring device. The monomer mixture solution may also contain other additives or components known in the art, if necessary. Examples of such additives and components include, but are not limited to, a diluent, a stabilizer, a dye, a pigment, an antimicrobial compound, and a mold releasing agent.

The polymerization initiator may be set appropriately according to the properties of the monomers used, and are not particularly limited. Examples of the polymerization initiator include peroxide type polymerization initiators such as lauroyl peroxide, cumene hydroperoxide and benzoyl peroxide, which are a common radical polymerization initiator; and azo type polymerization initiators such as azobis(dimethylvaleronitrile) and azobisisobutyronitrile. The polymerization initiator may be used either individually or in combination of two or more of those listed above. The amount of the polymerization initiator added is not particularly limited, as long as the amount can achieve the copolymerization reaction of monomers. Examples of the amount include preferably 10 ppm to 7,000 ppm relative to the total amount of monomer components.

The step (2) of obtaining the copolymer may be carried out by placing the monomer mixture solution in a molding die consisting of a pair of male and female molds that are made of metal, glass, plastic or other material and that are used to produce a contact lens by the cast mold method, and sealing and subjecting the molding die containing the monomer mixture solution to copolymerization reaction in a thermostatic bath or other device in which the temperature is raised in stages or continuously in the range of 25 °C to 120 °C for 5 hours to 120 hours. The copolymerization reaction may be conducted so as to induce radical polymerization. The copolymerization reaction may be copolymerization reaction by heating, copolymerization reaction using light ray such as ultraviolet ray, electron beam and gamma ray as well as copolymerization reaction by solution polymerization to add a solvent such as water and an organic solvent to the monomer mixture solution.

The step (3) of obtaining the hydrogel may be carried out by cooling the molding die subjected to the copolymerization reaction to room temperature and then detaching a copolymer from the molding die, cutting and/or polishing the resulting copolymer as necessary followed by hydrating and swelling the copolymer to obtain a hydrogel. Examples of the liquid (swelling solution) used include water, saline, and an isotonic buffer solution. The hydration swelling treatment may be preferably performed by immersing the copolymer in the swelling solution warmed to 60 °C to 100 °C for a certain period of time to put the copolymer in the swollen state. In the hydration swelling treatment, it is preferable to remove unpolymerized monomers contained in the polymer.

The hydrogel after hydration and swelling may contain about 40 wt% to about 80 wt% copolymer (polymer). In other words, the hydrogel base material may account for about 40 wt% to 80 wt% of the hydrogel. The remaining components of the hydrogel are typically composed of the liquid component (e.g., water) serving to hydrate the hydrogel base material.

The step (4) of obtaining the drug-bound hydrogel may be carried out by bringing the hydrogel obtained by the step (3) into contact with the drug-bound amphiphilic linker and subjecting the mixture to dehydration-condensation reaction to bind the drug to the hydrogel base material via the amphiphilic linker.

Examples of the dehydration-condensation reaction include immersing the hydrogel in a phosphate buffer (reaction solution) with the drug-bound amphiphilic linker and a dehydration-condensation agent (e.g., amidation condensation agent) resolved, and then shaking the reaction solution containing the hydrogel at a temperature of 15 °C to 40 °C, preferably at room temperature, for 12 hours to 72 hours, preferably for 20 hours to 50 hours. While each content of the amphiphilic linker and dehydration-condensation agent in the reaction solution is not particularly limited, examples of the content include 0.1 μmol/mL to 10 μmol/mL. The content of the amphiphilic linker in the reaction solution is preferably almost equal to or greater than the amount of a moiety that can bind to the amphiphilic linker in the drug-bound hydrogel according to one embodiment of the present invention. Examples of the content (μmol) of the amphiphilic linker in the reaction solution include, relative to the unit amino group number (μmol) of the hydrogel according to one embodiment of the present invention, preferably an amount corresponding to 0.5 to 10 equivalents, more preferably an amount corresponding to 0.8 to 8 equivalents, still more preferably an amount corresponding to 1.0 to 5 equivalents, and even still

more preferably an amount corresponding to 1.0 to 3 equivalents.

The drug-bound hydrogel after the dehydration-condensation reaction is preferably washed with a fresh phosphate buffer.

[Use of drug-bound hydrogel]

The drug-bound hydrogel according to one embodiment of the present invention may be flexible as a hydrogel, transparent, and capable of contacting or releasing the drug to the subject body. Because of such properties, the drug-bound hydrogel according to one embodiment of the present invention is, for example, applicable to an ocular lens used on or within the eye, and is preferably used as a contact lens and an intraocular lens. The contact lens is hydrophilic and may be a soft or hard contact lens (i.e., rigid gas permeable (RGP)). However, the drug-bound hydrogel according to one embodiment of the present invention does not necessarily have to release the drug to the subject body since the desired therapeutic or preventive effect of the drug may be achieved by bringing the bound drug in contact with the subject body such as the eye tissue.

When applied as an ocular lens, the drug-bound hydrogel according to one embodiment of the present invention preferably has a thickness of 50 μm to 150 μm.

The drug-bound hydrogel according to one embodiment of the present invention preferably has the transparency to the extent that the hydrogel is rated as "o" by the transparency evaluation described in Examples below when the thickness of the central portion is about 70 μm.

The drug-bound hydrogel according to one embodiment of the present invention has the water content of preferably 10 wt% or more, more preferably 20 wt% to 70 wt%, and still more preferably 30 wt% and 50 wt%.

The present invention will now be described in further detail with reference to Examples, which are not intended to limit the present invention. The present invention may take various embodiments to the extent that the objectives of the present invention are achieved.

Examples

<u>Example 1. Method of producing idoxuridine-bound linker (V)</u>

1-1. Summary

As described below, the idoxuridine-bound linker (V) was produced using idoxuridine and compound (III) as starting materials according to the above scheme (1).

1-2. Synthesis of compound (III)-A

To 1.5 ml of dichloromethane solution containing 104 mg (0.22 mmol) of protected idoxuridine with one hydroxy group of idoxuridine protected, 143 mg (0.22 mmol) of compound (III) was added, and then 1.5 ml of 0.25 M 5-(ethylthio) -1H-tetrazole in acetonitrile solution was added dropwise to obtain a reaction solution. The reaction solution was subjected to reaction by stirring at room temperature for 18 hours to obtain a primary reaction solution. Then, 140 μl of decane solution containing tert-butyl hydroperoxide (0.67 mmol) was added to the primary reaction solution, and the mixture was subjected to reaction by stirring at room temperature for 3 hours to obtain a secondary reaction solution. Then, 30 mL of dichloromethane was added to the secondary reaction solution, and the mixture was washed sequentially with saturated sodium bicarbonate solution and saturated brine, followed by being dried using anhydrous sodium sulfate. After removing the solvent from the resulting dry product, the residue was purified by silica gel column chromatography to obtain 106 mg of compound (III)-A (46% yield).

The NMR spectrum of the obtained compound (III)-A was as follows:

$^1$H-NMR($CDCl_3$) δppm; 0.06(d,6H), 0.86(s,9H), 2.03(m,1H), 2.27(m,1H), 2.70(m,2H), 3.79(s,6H), 4.01(m,1H), 4.17-4.24(m,6H), 4.41(m,1H), 5.17(d,2H), 6.21(m,1H), 6.84(m,4H), 7.22-7.32(m,3H), 7.40(m,8H), 7.49(m,2H), 7.95(d,1H), 8.44(d,1H)

1-3. Synthesis of compound (III)-B

To the reaction solution obtained by stirring 227 mg (0.22 mmol) of compound (III)-A in 3% dichloroacetic acid/dichloromethane at room temperature for 1 hour, 40 mL of dichloromethane was added, and then the mixture was washed sequentially with 5% sodium hydrogen carbonate solution and saturated brine, followed by being dried using anhydrous sodium sulfate. After removing the solvent from the resulting dry product, the residue was purified by silica gel column chromatography to obtain 147 mg of compound (III)-B (92% yield).

The NMR spectrum of the obtained compound (III)-B was as follows:
$^1$H-NMR($CDCl_3$) δppm; 0.09(s,6H), 0.88(s,9H), 1.92(m,1H), 2.09(m,1H), 2.30(m,1H), 2.72(m,2H), 4.01(m,1H), 4.18-4.28(m,4H), 4.39(m,1H), 4.70(d,2H), 5.15(d,2H), 6.18(m,1H), 7.38(m,4H), 7.89(m,1H), 9.28(d,1H)

1-4. Synthesis of compound (III)-C

To 1 mL of pyridine solution containing 96 mg (0.13 mmol) of compound (III)-B, 3.3 mg (0.027 mmol) of 4-dimethylaminopyridine (DMAP) and 27 mg (0.27 mmol) of succinic anhydride were added, and the mixture was stirred overnight at room temperature. The reaction solution was concentrated under reduced pressure, and the residue was purified by silica gel column chromatography to obtain 71 mg of compound (III)-C (65% yield).
The NMR spectrum of the obtained compound (III)-C was as follows:
$^1$H-NMR($CDCl_3$) δppm; 0.08(d,6H), 0.88(s,9H), 2.05(m,1H), 2.29(m,1H), 2.69(m,6H), 4.04(m,1H), 4.17-4.28(m,4H), 4.40(m,1H), 5.14(m,4H), 6.15(m,1H), 7.32-7.38(m,4H), 7.93(d,1H), 10.11(br,1H)

1-5. Synthesis of idoxuridine-bound linker (V)

To 2 mL of tetrahydrofuran solution containing 33 mg (0.04 mmol) of compound (III)-C, 96 μL (0.096 mmol) of TBAF was added, and the mixture was stirred overnight at room temperature. The reaction solution was concentrated under reduced pressure, and the residue was purified by silica gel column chromatography to obtain 24 mg (67% yield) of tetrabutylammonium salt of idoxuridine-bound linker (V).
The NMR spectrum of the obtained idoxuridine-bound linker (V) was as follows:
$^1$H-NMR($CD_3OD$) δppm; 1.00(m,12H), 1.40(m,8H), 1.65(m,8H), 2.18(m,2H), 2.61(m,4H), 3.22(m,8H), 3.95(m,1H), 4.03(m,2H), 4.41(m,1H), 4.98(m,2H), 5.10(s,2H), 6.21(m,1H), 7.36(m,4H), 8.18(s,1H)

Example 2. Method of producing idoxuridine-bound hydrogel

2-1. Preparation of hydrogel (1)

To the monomer solution containing 89.78 wt% 2-hydroxyethyl methacrylate (HEMA), 4.75 wt% methacrylic acid (MAA), 0.47 wt% ethylene glycol dimethacrylate (EDMA) and 5.0 wt% N-(2-aminoethyl) methacrylamide hydrochloride (AEMAm), 3,500 ppm (external) azobisisobutyronitrile (AIBN) was added with respect to the total mass of monomers, and the materials were stirred and mixed to prepare a monomer mixture solution.
The resulting monomer mixture solution was placed in a contact lens-shaped molding die, and the temperature was raised in the range of 30 °C to 100 °C for the period of 12 hours to obtain a polymer. The resulting polymer was brought to room temperature, detached from the molding die, and immersed in an ethanol-containing saline solution and a saline solution at 70 °C for 1 hour each to hydrate and swell the polymer to obtain a hydrogel. The resulting hydrogel was immersed in a saline solution and subjected to steam sterilization under pressure at 121 °C for 30 minutes to obtain hydrogel (1).

2-2. Preparation of hydrogel (2) and hydrogel (3)

Hydrogels (2) and (3) were obtained in the same manner as with hydrogel (1), except that 2-aminoethyl methacrylate hydrochloride (AEM) and N-(3-aminopropyl) methacrylamide hydrochloride (APMAm) were used instead of N-(2-aminoethyl) methacrylamide hydrochloride (AEMAm), respectively.

2-3. Preparation of idoxuridine-bound hydrogel

The idoxuridine-bound linker (V) and the amidation condensation agent 4-(4,6-dimethoxy-1,3,5-triazin-2-yl)-4-methylmorpholinium chloride (DMT-MM) were added into 5.0 mL of PBS in an amount of 5.0 μmol (corresponding to 1.20 to 1.50 equivalents relative to the amino group number per one hydrogel sheet (μmol/sheet)), respectively, and the mixture was stirred at room temperature to obtain a reaction solution.
In the resulting reaction solution, one sheet of the hydrogel was immersed, and the mixture was subjected to condensation reaction by shaking at 150 rpm for 24 hours at room temperature. The hydrogel after the condensation reaction was washed by repeating the immersion in PBS solution for 24 hours at room temperature twice to obtain an idoxuridine-bound hydrogel.
Three sheets of the hydrogel were used for each of hydrogels (1) to (3) to obtain idoxuridine-bound hydrogels of Examples 1 to 3. The hydrogel of Comparative Example 1 was prepared in the same manner as with Examples, except that the reaction solution was prepared by adding 5.0 μmol of idoxuridine along with DMT-MM into PBS followed by stirring at

room temperature.

Example 3. Evaluation (1) for idoxuridine-bound hydrogel

The idoxuridine-bound hydrogel with the idoxuridine-bound linker (V) bound was evaluated for transparency in the following manner.

The idoxuridine-bound hydrogels of Examples 1 to 3 were immersed in PBS solution with the concave side up and photographed from above, and evaluated for transparency according to the following criteria:

o: No white areas were observed;
o/x: White areas were observed; and
x: No idoxuridine-bound hydrogel was formed.

After wiping off excess water from any one of the idoxuridine-bound hydrogels of Examples 1 to 3, the weight (W1) of the hydrogel in the moisture state was measured. Then, after drying the hydrogel in a dryer at 60 °C for 24 hours, the weight (W2) of the hydrogel in the dried state was measured, and the water content was calculated from the weights according to the following formula:

$$\text{Water content (wt\%)} = [(W1-W2)/W1]x100$$

The results of the transparency evaluation and water content evaluation of the idoxuridine-bound hydrogels of Examples 1 to 3 are shown in Table 1. Figure 1 shows photographs of the idoxuridine-bound hydrogels of Examples 1 to 3 taken from above against a black background. As a result, the idoxuridine-bound hydrogels of Examples 1 to 3 were rated as "o" for transparency while the water content was relatively high to be 40% or more, indicating that the idoxuridine-bound hydrogel had the almost same transparency and water content as with a normal contact lens. On the other hand, in Comparative Example 1, no hydrogel could be prepared because idoxuridine could not dissolve in PBS solution.

[Table 1]

| | | Example 1 | Example 2 | Example 3 | Comp. Ex. 1 |
|---|---|---|---|---|---|
| Composition | HEMA | 89.78 | 89.78 | 89.78 | 89.78 |
| | MAA | 4.75 | 4.75 | 4.75 | 4.75 |
| | EDMA | 0.47 | 0.47 | 0.47 | 0.47 |
| | AEMAm | 5.00 | - | - | 5.00 |
| | AEM | - | 5.00 | - | - |
| | APMAm | - | - | 5.00 | - |
| | Phosphate linker | Yes | Yes | Yes | None |
| | Hydrogel water content (%) | 42 | 45 | 43 | - |
| | Hydrogel transparency | ○ | ○ | ○ | × |

※HEMA : 2-hydroxyethyl methacrylate
MAA : methacrylic acid
EDMA : ethylene glycol dimethacrylate
AEMAm : N-(2-aminoethyl) methacrylamide hydrochloride
AEM : 2-aminoethyl methacrylate hydrochloride
APMAM : N-(3-aminopropyl) methacrylamide hydrochloride

In addition, the idoxuridine-bound hydrogels of Examples 1 to 3 could release the bound drug, idoxuridine.

Example 4. Evaluation (2) for idoxuridine-bound hydrogel

The reaction solution containing 5 equivalents of idoxuridine-bound linker (V) and 5 equivalents of DMT-MM for the amino group number in each one sheet of hydrogels (1) to (3) was prepared. In the resulting reaction solution, one sheet of the hydrogel was immersed, and the mixture was subjected to condensation reaction by shaking at 150 rpm for 24

hours at room temperature. The condensation reaction was carried out by using three sheets for each of hydrogel (1), hydrogel (2) and hydrogel (3) to obtain idoxuridine-bound hydrogels of Examples 4 to 6.

The amount of idoxuridine-bound linker (V) in the reaction solution before and after the condensation reaction was determined by HPLC. Table 2 shows the results of determining the residual amount of idoxuridine-bound linker (V) in the reaction solution before condensation reaction, the reaction solution after condensation reaction in Example 4, the reaction solution after condensation reaction in Example 5, and the reaction solution after condensation reaction in Example 6, compared to the control (the solution containing only idoxuridine-bound linker (V)).

[Table 2]

| Sample solution | Residual amount (%) | Ave | SD | RSD |
|---|---|---|---|---|
| Control | - | - | - | - |
| Reaction solution before condensation reaction | 98.3 | - | - | - |
| Reaction solution after condensation reaction in Example 4 | 85.1 | 86.1 | 1.02 | 1.2 |
| | 86.1 | | | |
| | 87.1 | | | |
| Reaction solution after condensation reaction in Example 5 | 84.4 | 83.8 | 0.93 | 1.1 |
| | 84.3 | | | |
| | 82.7 | | | |
| Reaction solution after condensation reaction in Example 6 | 85.9 | 84.6 | 1.16 | 1.4 |
| | 83.6 | | | |
| | 84.2 | | | |

As shown in Table 2, the residual amount of idoxuridine-bound linker (V) in each reaction solution after condensation reaction in Examples 4 to 6 was within the range of 83.8% to 86.1% compared to the reaction solution before condensation reaction. This indicates that the idoxuridine-bound linker (V) in the reaction solution was bound to each hydrogel by highly efficient condensation reaction to produce the idoxuridine-bound hydrogels of Examples 4 to 6.

Thus, it was found that the idoxuridine-bound hydrogels of Examples 4 to 6 had the drug idoxuridine bound and could release idoxuridine when subjected to hydrolysis.

The results indicate that the idoxuridine-bound hydrogel is transparent and useful as a contact lens and other product that hold idoxuridine and treat diseases of the ocular tissue.

Industrial applicability

The drug-bound hydrogel according to one embodiment of the present invention can be used as a contact lens or any other product because the hydrogel can have both flexibility and transparency. In addition, the drug-bound hydrogel according to one embodiment of the present invention can contact or release the drug to the subject body, so that the hydrogel can be used as a medical device for the purpose of treating diseases of the ocular tissue, thereby contributing to the health and welfare of living individuals.

Cross-reference of related applications

The present application claims the benefit of priority to Japanese Patent Application No. 2023-053567, filed on March 29, 2023, the disclosure of which is incorporated herein by reference in its entirety. In addition, the disclosure of all the documents described herein including Patent Documents 1 and 2 is incorporated herein by reference in its entirety.

## Claims

1. A drug-bound hydrogel, wherein the drug is at least one drug selected from the group consisting of nucleic acid and nucleic acid analog, and wherein the drug is bound to at least one portion selected from the group consisting of the surface portion and interior portion of base material of the hydrogel via an amphiphilic linker.

**2.** The drug-bound hydrogel according to claim 1, wherein the amphiphilic linker is a phosphate linker represented by the following general formula (I)

[Chemistry 1]

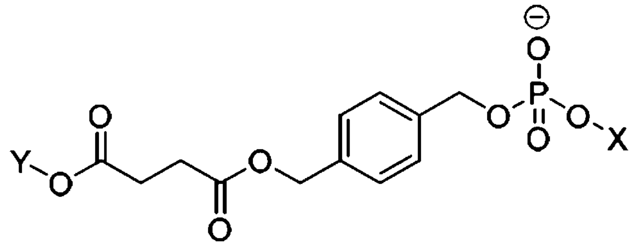

(I)

(wherein X represents a drug binding site and Y represents a hydrogel base material binding site)
, or
a phosphate linker represented by the following general formula (II)

[Chemistry 2]

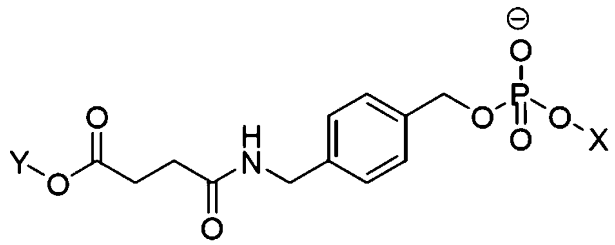

(II)

(wherein X represents a drug binding site and Y represents a hydrogel base material binding site).

**3.** The drug-bound hydrogel according to claim 1 or 2, wherein the drug is at least one nucleic acid analog selected from the group consisting of idoxuridine, acyclovir, abacavir, emtricitabine, zidovudine, lamivudine, vidarabine and cidofovir.

Figure 1

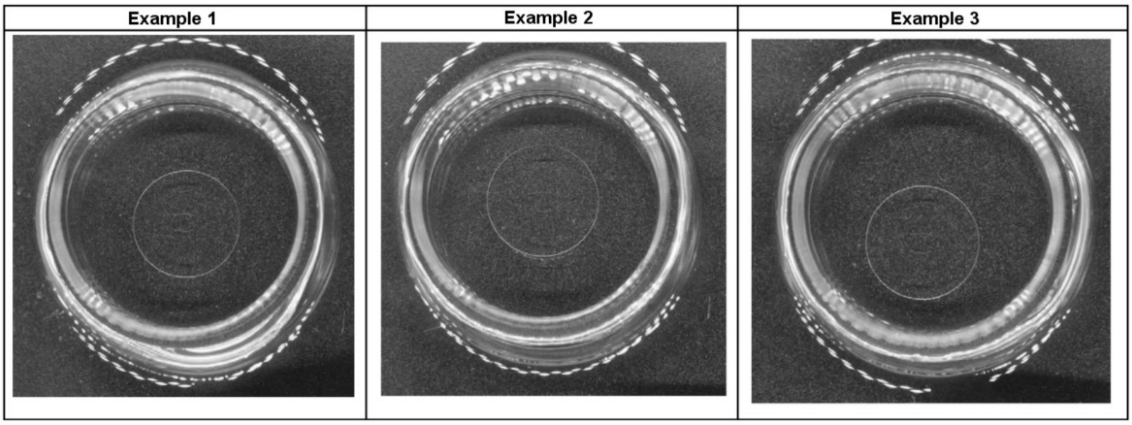

## INTERNATIONAL SEARCH REPORT

International application No.
**PCT/JP2024/011836**

**A. CLASSIFICATION OF SUBJECT MATTER**

***A61K 47/69***(2017.01)i; ***A61K 9/10***(2006.01)i; ***A61K 31/52***(2006.01)i; ***A61K 31/513***(2006.01)i; ***A61K 31/522***(2006.01)i; ***A61K 31/7072***(2006.01)i; ***A61K 47/58***(2017.01)i; ***A61K 48/00***(2006.01)i; ***A61P 27/02***(2006.01)i; ***A61P 29/00***(2006.01)i; ***A61P 31/12***(2006.01)i; ***C08F 8/40***(2006.01)i; ***C08F 20/22***(2006.01)i
FI: A61K47/69; C08F20/22; C08F8/40; A61K31/7072; A61K31/513; A61K31/522; A61K31/52; A61K48/00; A61P27/02; A61P31/12; A61P29/00; A61K9/10; A61K47/58

According to International Patent Classification (IPC) or to both national classification and IPC

**B. FIELDS SEARCHED**

Minimum documentation searched (classification system followed by classification symbols)

A61K47/69; A61K9/10; A61K31/52; A61K31/513; A61K31/522; A61K31/7072; A61K47/58; A61K48/00; A61P27/02; A61P29/00; A61P31/12; C08F8/40; C08F20/22

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2018-58898 A (NATIONAL UNIVERSITY CORPORATION GUNMA UNIVERSITY) 12 April 2018 (2018-04-12)<br>claims, paragraphs [0008], [0011]-[0034], examples | 1, 3<br>2 |
| X<br>A | JP 2021-169446 A (PEGAVISION CORP.) 28 October 2021 (2021-10-28)<br>claims, paragraphs [0028]-[0049], [0063]-[0075] | 1, 3<br>2 |
| X<br>A | JP 2008-220823 A (SEED CO., LTD.) 25 September 2008 (2008-09-25)<br>claims, paragraphs [0017]-[0018], examples | 1<br>2, 3 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/011836**

**C. DOCUMENTS CONSIDERED TO BE RELEVANT**

| Category* | Citation of document, with indication, where appropriate, of the relevant passages | Relevant to claim No. |
|---|---|---|
| X<br><br>A | JP 2023-507086 A (AMFERIA AB) 21 February 2023 (2023-02-21)<br>claims, paragraph [0055] | 1, 3<br><br>2 |
| A | WO 2020/116640 A1 (THE UNIVERSITY OF TOKYO) 11 June 2020 (2020-06-11)<br>entire text | 1-3 |
| A | JP 2021-512892 A (THE UNITED STATES OF AMERICA, AS REPRESENTED BY THE SECRETARY, DEPARTMENT OF HEALTH AND HUMAN SERVICES) 20 May 2021 (2021-05-20)<br>entire text | 1-3 |

Form PCT/ISA/210 (second sheet) (July 2022)

**INTERNATIONAL SEARCH REPORT**
**Information on patent family members**

International application No.
**PCT/JP2024/011836**

| Patent document cited in search report | Publication date (day/month/year) | Patent family member(s) | Publication date (day/month/year) |
|---|---|---|---|
| JP 2018-58898 A | 12 April 2018 | JP 2015-117228 A | |
| JP 2021-169446 A | 28 October 2021 | US 2021/0315807 A1<br>claims, paragraphs [0033]-[0054], [0068]-[0080]<br>EP 3895693 A1 | |
| JP 2008-220823 A | 25 September 2008 | (Family: none) | |
| JP 2023-507086 A | 21 February 2023 | US 2023/0346876 A1<br>claims, paragraph [0063]<br>WO 2021/126063 A1<br>EP 4076549 A1 | |
| WO 2020/116640 A1 | 11 June 2020 | (Family: none) | |
| JP 2021-512892 A | 20 May 2021 | US 2020/0360296 A1<br>entire text<br>WO 2019/157381 A1<br>EP 3749288 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004307574 A
- WO 2015159942 A
- JP 2023053567 A